# EUROPEAN PATENT SPECIFICATION

(11) **EP 0 630 878 B1**
(45) Date of publication and mention of the grant of the patent: **11.03.1998**
(21) Application number: 94107244.9
(22) Date of filing: 09.05.1994
(51) Int. Cl.: C07C 37/20, C07C 37/86, C07C 39/16

(54) **Process for the production of bisphenol A**
Verfahren zur Herstellung von Bisphenol A
Procédé pour la préparation du bisphénol A

(30) Priority: 12.05.1993 JP 110150/93
(43) Date of publication of application: 28.12.1994
(73) Proprietor: IDEMITSU PETROCHEMICAL CO. LTD., Tokyo 100 (JP)
(72) Inventor: Kawamura, Kouji c/o Idemitsu Petrochem. Co., Ltd, Yamaguchi-ken (JP); Sakakibara, Yasuzo c/o Idemitsu Petrochem. Co. Ltd, Yamaguchi-ken (JP); Morita, Minoru c/o Tsukishima Kikai Co., Ltd, Chuo-ku Tokyo (JP); Kamijo, Yasuhiko c/o Tsukishima Kikai Co., Ltd, Chuo-ku Tokyo (JP)
(74) Representative: VOSSIUS & PARTNER

(56) References cited:
- EP-A- 0 210 366
- EP-A- 0 332 203
- EP-A- 0 486 109
- US-A- 4 354 046

## Description

The present invention relates to an improved process for producing 2,2-bis(4-hydroxyphenyl)propane (hereinafter referred to as "bisphenol A"). More particularly, it relates to a process for producing bisphenol A in high yield by efficiently recovering bisphenol A through crystallization from the mother liquor of the reaction mixture obtained in the condensation reaction of phenol and acetone.

It is well known that bisphenol A is an important compound as a starting raw material for epoxy resins or engineering plastics such as polycarbonate resins and polyarylate resins, and accordingly, the demand thereof tends to expand year by year.

Bisphenol A is produced by the condensation reaction of excess phenol with acetone in the presence of an acidic catalyst and, as the case may be, a sulfur compound as cocatalyst. The reaction product thus formed contains, in addition to the objective bisphenol A, excess unreacted phenol, a small amount of unreacted acetone, the catalyst, water formed by the reaction and other reaction by-products.

As the above-mentioned reaction by-products, mention may be made of 2-(2-hydroxyphenyl)-2-(4-hydroxyphenyl)propane (hereinafter abbreviated to "o,p'-isomer") as the main component, dimer of isopropenylphenol, trisphenol, polyphenol and undesirable colored substances. Bisphenol A, particularly when used as the starting raw material for polycarbonate resins, polyarylate resins or the like, is required to be highly pure and of high-quality without coloration.

A variety of investigations have heretofore been made on the process for producing high-quality bisphenol A.

EP-A-0 486 109 relates to a process for the production of a bisphenol with improved product purity and yield. The process comprises the steps of reacting a carbonyl compound with a stoichiometric excess of a phenolic compound, passing the reaction product mixture to a crystallization zone to produce a crystalline bisphenol and a mother liquor, contacting in an isomerization zone, the mother liquor with an acidic ion-exchange resin as isomerization catalyst, contacting the isomerization reaction product mixture with a guard bed and recovering the bisphenol from the thus treated isomerization reaction product mixture.

EP-A-0 210 366 relates to a process for making bisphenol A by feeding phenol and acetone into a condensation reactor. The reaction mixture is separated in a crystallizer to provide the recovery of bisphenol A and the recycling back to the condensation reactor of a mixture of phenol and bisphenol A isomers through the rearrangement reactor. The process comprises the improvement of diverting part of the acetone initial fed to the condensation reactor to the rearrangement reactor, whereby an improvement in overall acetone conversion is achieved while the product distribution of the condensation reactor effluent is substantially maintained.

EP-A-0 332 203 provides a method for preparing high-purity bisphenol A comprising a principal process and a sub-process by recovering in a high yield bisphenol A from the mother liquor from which the adduct of bisphenol A with phenol has been separated, simultaneously removing coloring substances and other impurities to reduce the amount thereof, as low as possible, which may be recycled to the principal reaction process thereby increasing the yield and purity of crystals of the adduct product in the principal process.

One of the known processes is a process which comprises the steps of subjecting excess phenol with acetone to condensation reaction in the presence of a catalyst to form a reaction mixture, removing the catalyst, water and part of unreacted phenol from the reaction product, cooling the residual liquid to crystallize bisphenol A as the adduct with phenol, separating the crystals from the solution containing the reaction by-products, and removing phenol from the aforesaid adduct to recover the objective bisphenol A. Various methods were proposed for separating phenol from the adduct of bisphenol A with phenol, such as decomposition, destillation, extraction and steam stripping.

In order to enhance the yield of bisphenol A as the objective product in the above-mentioned process, it is taken into consideration to recycle the mother liquor from which the adduct of the bisphenol A with phenol has been removed through the crystallization system. However, if the aforesaid mother liquor is recycled as it is through the crystallization system, impurities in the mother liquor are accumulated therein, inevitably requiring a part of the mother liquor to be withdrawn outside the reaction system. The aforestated procedure leads to the loss of objective bisphenol A because a considerable amount of the mother liquor has to be taken out of the reaction system in view of the high concentration of the o,p'-isomer in the liquor.

In addition, in the case of recycling the mother liquor through the reaction system, although the isomerization from o,p'-isomer to p,p'-isomer, that is, the objective bisphenol A takes place, there are brought about unfavorable circumstances such as accelerated deterioration of the catalyst and increase in the amounts of the colored substances.

The primary object of the present invention is to provide a process for producing bisphenol A as the objective product in high yield by efficiently recovering bisphenol A through crystallization from the mother liquor of the reaction mixture obtained in the condensation reaction of phenol and acetone.

This object could surprisingly be attained by bringing the above-mentioned mother liquor into contact with an acid catalyst to proceed with the isomerizing treatment, and thereafter recycling the mother liquor thus treated through the crystallization system.

Specifically, the present invention provides a process for producing bisphenol A comprising the steps of subjecting phenol and acetone to a condensation reaction in the presence of an acid catalyst to form a reaction mixture, crystallizing an adduct of bisphenol A and phenol from the reaction mixture, separating the crystallized adduct from the reaction mixture and separating the phenol from the adduct which process comprises further the steps of bringing the reaction mixture in the form of a liquid from which the adduct has been removed into contact with an acid catalyst to effect isomerisation and recycling the mixture through the crystallization system.

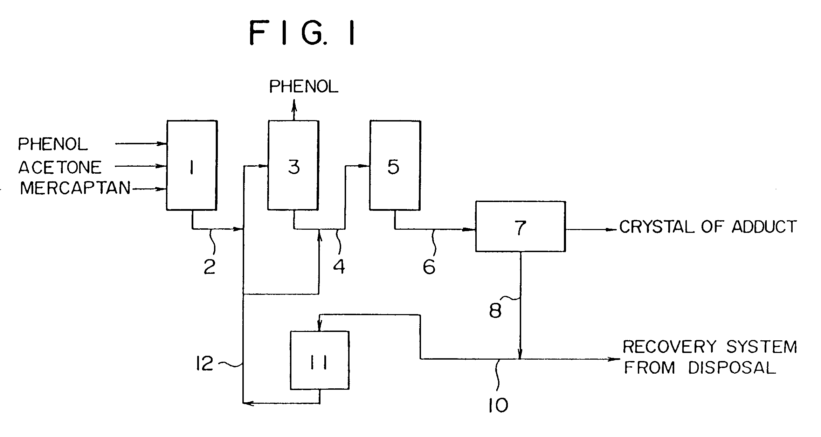

Fig. 1 is a block flow diagram showing one example of a production process for bisphenol A to carry out the present invention wherein the symbols 1,3,5,7 and 11 denote the following:
1 : Reactor, 3 : Concentration step, 5 : Crystallization step 7 : Solid-liquid separation step, 11 : Isomerization step

In the process according to the present invention, first of all excess phenol and acetone are subjected to a condensation reaction to form bisphenol A in the presence of an acid catalyst, which is selected for the above purpose from an acid such as hydrochloric acid and sulfuric acid and a cation-exchange resin. There is generally used as the cation-exchange resin, the one having a styrene/divinylbenzene copolymer in the skeleton. In addition, for the purpose of, as desired, enhancing the selectivity or the rate of reaction, there is usable as a cocatalyst, a mercaptan such as ethyl mercaptan, methyl mercaptan and n-octyl mercaptan.

Moreover, a phenol/acetone molar ratio is selected in the range of usually 3 to 30, preferably 5 to 20. A molar ratio thereof exceeding 30 results in the tendency to lower the rate of reaction, whereas that less than 3 leads to the tendency to lower the selectivity to the objective bisphenol A (p,p'-isomer). On the other hand, in the case of employing a cation-exchange resin as the catalyst, the reaction temperature is selected in the range of usually 45 to 110°C. A reaction temperature in that case lower than 45°C results in lowered rate of reaction and, as the case may be, a fear of solidification of the reactants, whereas that higher than 110°C brings about lowered selectivity to the objective bisphenol A (p,p'-isomer) as well as disadvantage from the viewpoint of the heat resistance of the ion exchange resin.

The reaction product obtained in the above-mentioned manner is preferably concentrated according to the publicly known method after the removal of the acid catalyst and prior to crystallization. In the case of employing a reactor containing cation exchange resin in the form of fixed bed, the catalyst need not be removed. The concentration is not specifically limited in its conditions, but is carried out under the conditions including a temperature in the range of 100 to 170°C and a pressure in the range of 5320 to 66500 Pa (40 to 500 torr). A concentration temperature lower than 100°C necessitates a high vacuum, while that higher than 170°C necessitates the removal of excessive heat in the successive crystallization step. The concentration of bisphenol A in the concentrated residue is preferably in the range of 20 to 40% by weight. A concentration thereof lower than 20% by weight leads to lowered yield of the objective bisphenol A, whereas that higher than 40% by weight causes difficulty in the transfer of the slurry after crystallization step.

Crystallization of the adduct of bisphenol A with phenol from the concentrated residue is carried out usually by the conventional cooling crystallization method. The crystallization temperature is in the range of usually 40 to 70°C. A crystallization temperature in that case lower than 40°C involves a fear of increasing viscosity of the crystallization liquid or causing solidification thereof, while that higher than 70°C unfavorably increases the dissolution loss of bisphenol A.

The adduct of bisphenol A with phenol which has been crystallized in the aforestated manner is separated from the mother liquor by means of a known method such as the method using a vacuum filter or a centrifuge. The mother liquor thus separated usually contains p,p'-isomer and o,p'-isomer in a p,p'-isomer/o,p'-isomer ratio of 3 to 4 and is transferred to isomerization step to isomerize the o,p'-isomer into the p,p'-isomer by bringing the mother liquor into contact with an acid catalyst, preferably a cation-exchange resin of the sulfonic acid type. In this step, a part of the mother liquor is usually taken out of the isomerization system in order to suppress the accumulation of impurities contained in the liquor. There is usually employed in the isomerization reaction, a reactor packed with a generally well-known cation-exchange resin of the sulfonic acid type as the catalyst to proceed with isomerization treatment under atmospheric pressure at a temperature in the range of 45 to 110°C. By the isomerization treatment, the p,p'-isomer/o,p'-isomer ratio in the mother liquor is usually raised to 7 to 8.

The cation-exchange resin of the sulfonic acid type to be used in the isomerization is not specifically limited provided that it is a strongly acidic cation-exchange resin having a sulfonic acid group, but is exemplified by sulfonated styrene/divinylbenzene copolymer, sulfonated crosslinked styrene polymer, phenol-formaldehyde-sulfonic acid resin and benzene-formaldehyde-sulfonic acid resin. Any of the above-exemplified resins may be used alone or in combination with at least one other resin.

The isomerization temperature lower than 45°C gives rise to a fear of bisphenol A crystallization, whereas that higher than 110°C causes the problem with the heat resistance of the catalyst. The reaction is not specifically limited in the process but is preferably carried out in continuous reaction process with fixed bed or batch-wise reaction process. In the case of effecting continuous reaction with fixed bed, a liquid hourly space velocity (LHSV) is selected in the range of usually 0.1 to 10 Hr⁻¹, preferably 0.5 to 5 Hr⁻¹. A LHSV in the above reaction less than 0.1 Hr⁻¹ results in an increase in the amount of the by-products, while that more than 10 Hr⁻¹ brings about a tendency to lower the conversion efficiency.

The isomerization-treated liquid is recycled through the crystallization system. When the concentration of bisphenol A in the liquid is low, it is preferable that the liquid together with the main reaction liquid be fed to the concentration step to distil away phenol and regulate the concentration of bisphenol A and then fed to the crystallization system. When the concentration of bisphenol A in the liquid is high, the liquid may be fed directly to the crystallization system. The p,p'-isomer increased in the content thereof through the isomerization treatment is recovered in the form of crystal of the adduct of bisphenol A with phenol in the crystallization system.

The crystalline adduct of bisphenol A with phenol which has been separated by crystallization in the crystallization step is cleaned usually with phenol and thereafter the phenol contained in the adduct is removed to recover the objective bisphenol A. The method for recovering bisphenol A is not specifically limited but may be the publicly known recovery method, preferably decomposition method. In the decomposition method, phenol is removed and bisphenol A is recovered by decomposing the adduct under a reduced pressure of usually in the range of 2660 to 19950 Pa (20 to 150 torr). at a reaction temperature of 130 to 200°C, preferably 150 to 180°C. The decomposition temperature exceeding 200°C brings about unfaborable results such as an increase in the amounts of impurities in the objective bisphenol A arising from the decomposition of bisphenol A itself and coloration.

The bisphenol A obtained by decomposing the adduct of bisphenol A with phenol is turned into the objective high-quality bisphenol by almost completely removing the residual phenol through steam stripping method or like method.

Fig. 1 is a block flow diagram showing one example of the production process for bisphenol A to carry out the present invention. As illustrated in Fig. 1, the reaction mixture 2 coming out from the reactor 1 together with the liquid to be isomerization treated 12 coming from the isomerization step 11 is transferred to the concentration step 3; the concentrate 4 concentrated therein is introduced in the crystallization step 5, where the adduct of bisphenol A with phenol is crystallized; the slurry 6 containing the crystalline adduct is sent to the solid-liquid separation step 7, where the slurry 6 is separated into the crystalline adduct and mother liquor 8, a part of which is withdrawn outside the reaction system and the remainder 10 is transferred to the isomerization step 11 for the isomerization thereof; and the isomerization treated liquid 12 together with the reaction mixture 2 are fed to the concentration step.

According to the process of the present invention in which the mother liquor of the reaction mixture obtained through the condensation reaction of phenol and acetone is subjected to isomerization treatment and thereafter recycled through the crystallization system, bisphenol A is efficiently recovered from the mother liquor and bisphenol A as the objective product is obtained in high yield.

In the following, the present invention will be described in more detail with reference to the example.

### Example 1

The reaction mixture obtained by condensation reaction of phenol and acetone was concentrated and crystallized at 45°C to separate the crystalline adduct of the resultant bisphenol A with phenol and take out the mother liquor having a p,p'-isomer/o,p'-isomer ratio of 3.

The mother liquor was fed into an isomerization reactor packed with a cation-exchange resin of the sulfonic acid type to carry out isomerization treatment thereof under the conditions including a reactor inlet temperature of 75°C and a LHSV of 1.0 Hr⁻¹. As a result, the p,p'-isomer/o,p'-isomer ratio after the isomerization was raised as high as 8.

Subsequently, the isomerization treated liquid was recycled and fed to a concentration tower to recover bisphenol A by crystallization. As a result, the recovery rate of bisphenol A thus isomerized was 25% by weight higher than the recovery rate of bisphenol A formed by recycling the mother liquor as such through the crystallization system without isomerization.

## Claims

1. A process for producing 2,2-bis(4-hydroxyphenyl)propane comprising the steps of subjecting phenol and acetone to a condensation reaction in the presence of an acid catalyst to form a reaction mixture, crystallizing an adduct of 2,2-bis(4-hydroxyphenyl)propane and phenol from the reaction mixture, separating the crystallized adduct from the reaction mixture and separating the phenol from the adduct; said process comprising further the steps of bringing the reaction mixture in the form of a liquid from which the adduct has been removed into contact with an acid catalyst to effect isomerisation and recycling the mixture through the crystallization system.

2. The process according to Claim 1 wherein the acid catalyst to be employed in the isomerizing treatment is a cation-exchange resin of the sulfonic acid type.

3. The process according to Claim 2 wherein the cation-exchange resin of the sulfonic acid type is a strongly acidic cation-exchange resin having a sulfonic acid group selected from the group consisting of sulfonated styrene/divinylbenzene copolymer, sulfonated crosslinked styrene polymer, phenol-formaldehyde-sulfonic acid resin and benzene-formaldehyde-sulfonic acid resin.

4. The process according to any of Claims 1 to 3 wherein the isomerizing treatment is effected at a temperature in the range of 45 to 110°C.

5. The process according to any of Claims 1 to 4 wherein the isomerizing treatment is effected by continuous reaction process with fixed bed at a liquid hourly space velocity (LHSV) of 0.1 to 10 Hr⁻¹.

6. The process according to any of Claims 1 to 5 which further comprises a step of concentrating the reaction mixture prior to the crystallization step.

7. The process according to Claim 6 where the step of concentrating the reaction mixture is effected at a temperature in the range of 100 to 170°C under a pressure of 5320 to 66500 Pa (40 to 500 torr).

8. The process according to Claim 6 or 7 wherein the reaction mixture is concentrated to 20 to 40% by weight.

## Patentansprüche

1. Verfahren zur Herstellung von 2,2-Bis(4-hydroxyphenyl)propan, umfassend die Schritte: Kondensieren von Phenol und Aceton in Gegenwart eines sauren Katalysators, wodurch ein Reaktionsgemisch erzeugt wird, Kristallisieren eines Addukts aus 2,2-Bis(4-hydroxyphenyl)propan und Phenol aus dem Reaktionsgemisch, Abtrennen des kristallisierten Addukts aus dem Reaktionsgemisch und Abtrennen des Phenols aus dem Addukt; wobei das Verfahren ferner die Schritte umfaßt: in Kontakt bringen des Reaktionsgemischs in Form einer Flüssigkeit, aus der das Addukt entfernt wurde, mit einem sauren Katalysator, um Isomerisierung zu bewirken und Rückführung des Gemisches durch das Kristallisationssystem.

2. Verfahren nach Anspruch 1, wobei der bei der Isomerisierungsbehandlung zu verwendende saure Katalysator ein Kationenaustauscherharz vom Sulfonsäuretyp ist.

3. Verfahren nach Anspruch 2, wobei das Kationenaustauscherharz vom Sulfonsäuretyp ein stark saures Kationenaustauscherharz mit einer Sulfonsäuregruppe, ausgewählt aus sulfoniertem Styrol/Divinylbenzolcopolymer, sulfoniertem, vernetzem Styrolpolymer, Phenol-Formaldehyd-Sulfonsäureharz und Benzol-Formaldedyd-Sulfonsäureharz, ist.

4. Verfahren nach einem der Ansprüche 1 bis 3, wobei die Isomerisierungsbehandlung bei einer Temperatur im Bereich von 45 bis 110°C durchgeführt wird.

5. Verfahren nach einem der Ansprüche 1 bis 4, wobei die Isomerisierungsbehandlung mittels eines kontinuierlichen Reaktionsverfahrens mit Festbett bei einer Raumgeschwindigkeit der Flüssigkeit pro Stunde (LHSV) von 0,1 bis 10 h⁻¹ durchgeführt wird.

6. Verfahren nach einem der Ansprüche 1 bis 5, ferner umfassend einen Schritt des Aufkonzentrierens des Reaktionsgemischs vor dem Kristallisationsschritt.

7. Verfahren nach Anspruch 6, in dem der Schritt des Aufkonzentrierens des Reaktionsgemischs bei einer Temperatur im Bereich von 100 bis 170°C unter einem Druck von 40 bis 500 Torr durchgeführt wird.

8. Verfahren nach Anspruch 6 oder 7, wobei das Reaktionsgemisch auf 20 bis 40 Gewichts-% aufkonzentriert wird.

## Revendications

1. Procédé de production de 2,2-bis(4-hydroxyphényl)propane comprenant les étapes consistant à soumettre du phénol et de l'acétone à une réaction de condensation en présence d'un catalyseur d'acide pour former un mélange réactionnel, à cristalliser un produit d'addition de 2,2-bis(4-hydroxyphényl)propane et de phénol dans le mélange réactionnel, à séparer le produit d'addition cristallisé du mélange réactionnel et à séparer le pnénol du produit d'addition, ledit procédé comprenant de plus les étapes consistant à amener le mélange réactionnel sous la forme d'un liquide dont le produit d'addition a été séparé au contact d'un catalyseur d'acide pour effectuer l'isomérisation et à recycler le mélange par le système de cristallisation.

2. Procédé suivant la revendication 1, dans lequel le catalyseur d'acide à utiliser dans le traitement d'isomérisation est une résine échangeuse de cations du type acide sulfonique.

3. Procédé suivant la revendication 2, dans lequel la résine échangeuse de cations du type acide sulfonique est une résine échangeuse de cations fortement acide comportant un groupe acide sulfonique choisie dans le groupe comprenant un copolymère de styrène/divinyl benzène sulfoné, un polymère de styrène réticulé sulfoné, une résine de phénol-formaldéhyde-acide sulfonique et une résine de benzène-formaldéhyde-acide sulfonique.

4. Procédé suivant l'une quelconque des revendications 1 à 3, dans lequel le traitement d'isomérisation est effectué à une température dans l'intervalle de 45 à 110°C.

5. Procédé suivant l'une quelconque des revendications 1 à 4, dans lequel le traitement d'isomérisation est effectué par un procédé réactionnel continu avec un lit fixe à une vitesse spatiale horaire liquide (LHSV) de 0,1 à 10 hre⁻¹.

6. Procédé suivant l'une quelconque des revendications 1 à 5, qui comprend de plus une étape de concentration du mélange réactionnel avant l'étape de cristallisation.

7. Procédé suivant la revendication 6, dans lequel l'étape de concentration du mélange réactionnel est effectuée à une température dans l'intervalle de 100 à 170°C sous une pression de 40 à 500 torrs.

8. Procédé suivant l'une ou l'autre des revendications 6 et 7, dans lequel le mélange réactionnel est concentré à de 20 à 40 % en poids.
